(19) Europäisches Patentamt / European Patent Office / Office européen des brevets

(11) **EP 4 372 623 A1**

(12) **EUROPEAN PATENT APPLICATION**

(43) Date of publication:
**22.05.2024   Bulletin 2024/21**

(21) Application number: **22207547.5**

(22) Date of filing: **15.11.2022**

(51) International Patent Classification (IPC):
**G06N 3/09** (2023.01)     **G16H 40/40** (2018.01)
**G06Q 10/20** (2023.01)

(52) Cooperative Patent Classification (CPC):
**G06N 3/09; G06Q 10/20; G16H 40/40;**
G06N 3/0475

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **PATIL, Meru Adagouda**
  **Eindhoven (NL)**
• **AGARWAL, Vani**
  **Eindhoven (NL)**
• **NAIK, Sarif Kumar**
  **5656AG Eindhoven (NL)**

(74) Representative: **Philips Intellectual Property &
Standards
High Tech Campus 52
5656 AG Eindhoven (NL)**

(54)   **RESOLVING PROBLEMS WITH MEDICAL DEVICES**

(57)   Proposed are schemes, solutions, concepts, designs, methods and systems pertaining to assessing solutions for diagnosis and fixing medical devices. Specifically, concepts for generating a machine learning model adapted to output a solution-component graph representing possible actions to be performed on components of a medical device to solve a problem with the medical device are proposed. Solution-component graphs for (new and previously seen) problems with the medical device can be generated, and can be compared to competencies of one or more individuals in order to ascertain the likelihood that the individual(s) can perform the actions to solve the problem. Thus, embodiments of the invention provide an efficient and accurate means for managing the process of diagnosing and fixing a medical device.

FIG. 3

EP 4 372 623 A1

**Description**

FIELD OF THE INVENTION

The present invention relates to the field of assessing problems with medical devices, and more particularly determining possible actions to be performed on components of a medical device to solve a problem with the medical device.

BACKGROUND OF THE INVENTION

Healthcare facilities usually have multiple medical devices spread across many different departments. Typically, these medical devices are from multiple different Original Equipment Manufacturers (OEMs), due to pricing, performance, and availability considerations. Each OEM sells and services many types of medical device

For day-to-day operation and maintenance of these devices, healthcare facilities use a few resources. One resource is a team/individual in each department who manages and operates devices within that department. Another resource is a central biomedical engineering team with responsibility for maintenance of all medical devices within the healthcare facility.

Furthermore, each OEM often provides a dedicated service team consisting of Field Service Engineers (FSEs) who are trained for maintenance of medical devices provided by the OEM. FSEs are generally trained to maintain, service and fix a range of medical devices as part of their basic training session, and are often specialized in a small number of medical devices.

In practice, when an issue with a medical device arises, the central bio-medical engineering team of the healthcare facility is notified by the respective department. Often, the central team will then contact the OEMs central helpline. At the OEM side, the central helpline team takes each reported issue and assigns to an appropriate FSE. This practice is particularly sub-optimal when more than one medical device (of one or more department) has a similar issue at same time, as the healthcare facility flags an individual problem with the OEM for each of the problems.

Of course, the central bio-medical engineering can solve certain issues associated with the medical devices without help from the OEM. Every bio-medical engineer has certain competencies based on which they can provide inputs to solve an issue with the medical device. There may be certain issues which can be solved with the help of remote guidance from the OEM. For other issues, FSEs may have to visit the healthcare facility to resolve the issue. The process of identifying who is equipped to resolve the issue, and communicating the issue inherently consumes a lot of time, and therefore increases the downtime of the medical devices.

Thus, there exists a present need for an efficient and accurate means for categorizing problems with medical devices, and identifying parties who are best equipped to solve them.

SUMMARY OF THE INVENTION

The invention is defined by the claims.

According to examples in accordance with an aspect of the invention, there is provided a method for training a machine learning model adapted to output, based on inputting a problem with a medical device, a solution-component graph representing possible actions to be performed on components of the medical device to solve the problem, the method comprising:

obtaining solution data describing sequences of previous actions performed to solve a plurality of previous problems with the medical device;
obtaining a component graph describing interactions between a plurality of components of the medical device;
processing the solution data and the component graph to generate a plurality of historic solution-component graphs; and
training the machine learning model using a training algorithm configured to receive an array of training inputs and respective known outputs, wherein a training input comprises one of the previous problems, and wherein a respective known output comprises the historic solution-component graph associated with the previous problem.

Proposed are concepts for training of a machine learning model that can generate/output solution-component graphs for (previously unseen) problems with a medical device. Such solution-component graphs represent possible actions to be performed on components on a medical device to diagnose and/or fix the problem with the medical device.

This is achieved by producing a training set of solution-component graphs based on data of previous problems with a (same type of) medical device, and the actions taken to solve them. This (historic) solution data is combined with a component graph (representing interactions between components) in order to produce the historic solution-component graphs. Accordingly, the training set of historic solution-component graphs are used as known outputs, with corresponding problems being known inputs, such that the machine learning model may be trained.

Thus, there is provided a unique facility for producing solution-component graphs. Such graphs essentially describe a (potentially branching) sequence of possible actions that need to be performed on components (i.e. parts and modules) of the medical device in order to resolve a corresponding problem. As such, they describe a full range of possible actions that may need to be taken, which can be particularly useful when assessing who is best to resolve the problem.

By way of example, if a first party has one set of competencies, and a second party has another set of competencies, then the competencies can be compared to the possible actions described by generated solution-component graphs to determine which of the first and second party is best placed to resolve the problem. Indeed, a degree to which each party can solve the problem (and an extent to which collaboration may be required) could be determined in a simple manner from the solution-component graphs.

Moreover, machine learning models provide an effective means for producing outputs for previously unseen inputs. Thus, by leveraging a large amount of available solution data, the machine learning model may be adapted for accurate and useful production of solution-component graphs for previously unseen problems, which otherwise would typically require substantial amounts of time and effort from a knowledgeable and skilled technician.

In some embodiments, obtaining the plurality of solution data may comprise obtaining previous problem data describing historic issues with the medical device, obtaining previous action data describing actions performed to solve historic issues with the medical device, and processing the previous problem data and previous action data to generate the plurality of solution data.

It is often the case that aspects of the solution data (describing previous actions performed to solve a previous problem) are found in a variety of different sources. Thus, it may be necessary to obtain separate problem data and previous action data, and process such data (i.e. match the problems with the actions, potentially based on timestamps and/or labelling) in order to produce a full set of solution data. Thus, a large set of solution data may be produced, meaning more training data for the machine learning model. Consequentially, the machine learning model may be more accurate, particularly for previously unseen problems.

In some embodiments, processing the previous problems and sequences of previous actions may comprise analysing the previous problem data and previous action data to generate raw solution data, identifying similar solutions within the raw solution data based on the previous problem data and the previous action data, and generating the plurality of solution data based on the raw solution data and the identified similar solutions.

In other words, there may be a lot of redundant data in the previous problems and sequences of previous solutions. There may be very typical problems that have occurred many times in the past, or slightly different problems that have highly similar solutions/sequences of actions. Producing solution data having a lot of redundant data may result in many highly similar historical solution-component graphs for training of the machine learning model, which may be of limited benefit to the training of the model, while still requiring a lot of processing and time for the training process. Therefore, consolidating the data (i.e. merging similar problems/solutions, or deleting highly similar data) before the training stage may save time and reduce computational complexity of the method for training the machine learning model, while having limited impact on the utility of the model.

In some embodiments, the previous problem data may be obtained from problem statements and device logs associated with the medical device, and previous action data may be obtained from service histories associated with the medical device.

Some useful sources for the previous problem data and previous action data are problem statements, device logs, and service histories, which all describe the status of the device, and actions taken over time in the maintenance, servicing and fixing of the medical device. Thus, by using such sources, a large amount of training data may be generated.

In some embodiments, obtaining the component graph may comprise processing interaction data of each component of the medical device describing an interaction of said component with other components of the medical device, to generate the component graph. The interaction data may be obtained from design documents, design drawings, production drawings and service manuals associated with components of the medical device.

When a component graph is not pre-generated/already available, one method for producing is to compile interaction data of each component, and to process this data to generate the component graph. Design documents, design drawings, production drawings and service manuals provide a comprehensive list of interactions between components, such that a full understanding of the medical device may be obtained.

In some embodiments, generating each historic solution-component graph may comprise mapping one of the sequences of previous actions described by the solution data onto the interactions between the components of the medical device described by the component graph, and generating the historic solution-component graph based on the mapping.

Specifically, the mapping may be based on processing the sequences of previous actions and the components of the medical device using a natural language processing algorithm.

An effective method for producing solution-component graphs from component graphs and solution data is to map the previous actions onto interactions between the components. This essentially produces a list of components and movements between components needed to resolve a problem. This can be achieved automatically by matching similar words (i.e. by semantic matching) using natural language processing algorithms. Thus, an accurate historic solution-component graph can be generated using known methods in a simple manner that does not require excessive computation or time of a trained person.

In exemplary embodiments, the machine learning model may be a generative adversarial network model.

A generative adversarial network (GAN) is particularly adept at producing new information based on training data that is different. Thus, A GAN-based machine learning model may be particularly effective at producing solution-component graphs corresponding to previously unseen problems (i.e. problems not associated with solution data in the training set).

The method may further comprise retraining the machine learning model for a user. This is performed by obtaining user-specific previous problems describing historic issues with the medical device associated with the user, obtaining user-specific historic solution-component graphs associated with the user-specific previous problems, and retraining the machine learning model using a training algorithm configured to receive an array of training inputs and respective known outputs, wherein a training input comprises one of the user-specific previous problems, and wherein a respective known output comprises the user-specific historic solution-component graph associated with the user-specific previous problem.

Specific users (i.e. medical facilities) may have their own particular set of problems, and ways of providing solutions. In other words, different users may employ different sequences of actions to resolve similar problems (i.e. due to policy, trained practice, etc.). Thus, retraining the (generic) machine learning model based on user-specific data may enable the production of solution-component graphs for new problems experienced by the user that describe sequences of actions likely to be more appropriate for the user.

Thus, by retraining the machine learning model in this way, improved (i.e. more accurate for the user) problem-solution graphs may be generated by the machine learning model, improving a utility of such graphs.

According to another aspect of the invention, there is provided a method for generating a solution-component graph representing possible actions to be performed on components of the medical device to solve the problem, the method comprising:

> generating a machine learning model according to an embodiment of the invention;
> obtaining a problem describing an issue with the medical device; and
> acquiring the solution-component graph based on inputting problem to the generated machine learning model.

Thus, a problem-component graph for a new problem can be generated by leveraging the machine learning model generated according to other aspects of the invention. This is useful for determining how, and by who, the problem should/may be resolved. Further, this may be particularly useful when the problem is a previously unseen problem, in which it may be difficult to determine the actions required to resolve the problem and who may be best equipped to do so.

According to a further aspect of the invention, there is provided a method for generating a solution-component graph using a machine learning model trained to output, based on inputting a problem with a medical device, the solution-component graph representing possible actions to be performed on components of the medical device to solve the problem, the method comprising:

> obtaining a problem describing an issue with the medical device; and
> acquiring the solution-component graph based on inputting problem to the generated machine learning model.

According to yet another aspect of the invention, there is provided a method for determining the solvability of a problem with a medical device by a one or more individuals, the method comprising:

> generating a solution-component graph representing possible actions to be performed on components of the medical device to solve the problem according to an embodiment of the invention
> obtaining competency data describing an ability of the one or more individuals to perform a plurality of actions on the components of the medical device; and
> processing the historic solution-component graphs, the solution-component graph and the competency data to generate a solvability value indicative of a likelihood that the one or more individuals can solve the problem with the medical device.

A particular advantage enabled by the generation of solution-component graphs for a problem is the capability to then compare the generated solution-component graphs with historic solution-component graphs and capabilities of one or more individuals to determine whether (and/or to what extent) they may be able to solve the problem.

Thus, this embodiment of the invention provides a simple and automatic means for determining whether one or more individuals can solve the problem. This may be useful for the effective division of problem solving/resolution. For example, if the (new) problem is determined by the invention to be easily solvable, then a highly trained individual may not be required (saving time of the highly trained individual). Conversely, if it is determined that the (new) problem is complex, then notification of the problem may be quickly escalated to one or more individuals best equipped to resolve the problem. Accordingly, proposed concepts facilitate an improved speed of medical device problem resolution (i.e. a reduced downtime for medical devices), while reducing the amount of work required for highly trained technicians to partake in.

According to other aspects of the invention, there is provided a computer program comprising computer program code means adapted, when said computer program is run on a computer, to implement any foregoing embodiment of the invention.

According to a final aspect of the invention, there is provided a system for training a machine learning model adapted to output, based on inputting a problem with a medical device, a solution-component graph representing possible actions to be performed on components of the medical device to solve the problem, the system comprising:

a data interface configured to:

obtain solution data describing sequences of previous actions performed to solve a plurality of previous problems with the medical device; obtain a component graph describing interactions between a plurality of components of the medical device;

a processor configured to process the solution data and the component graph to generate a plurality of historic solution-component graphs; and a training module configured to train the machine learning model using a training algorithm configured to receive an array of training inputs and respective known outputs, wherein a training input comprises one of the previous problems, and wherein a respective known output comprises the historic solution-component graph associated with the previous problem.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

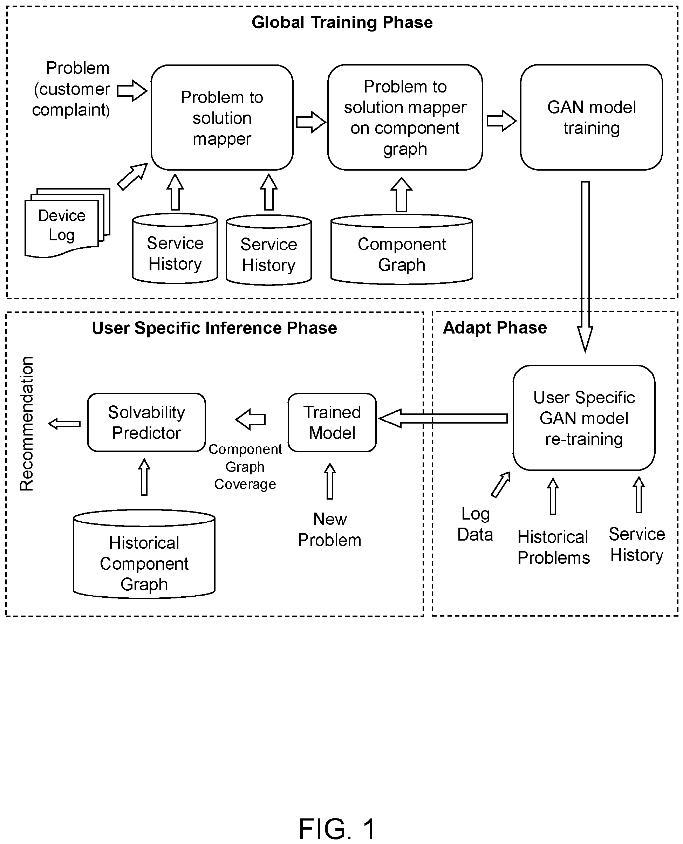

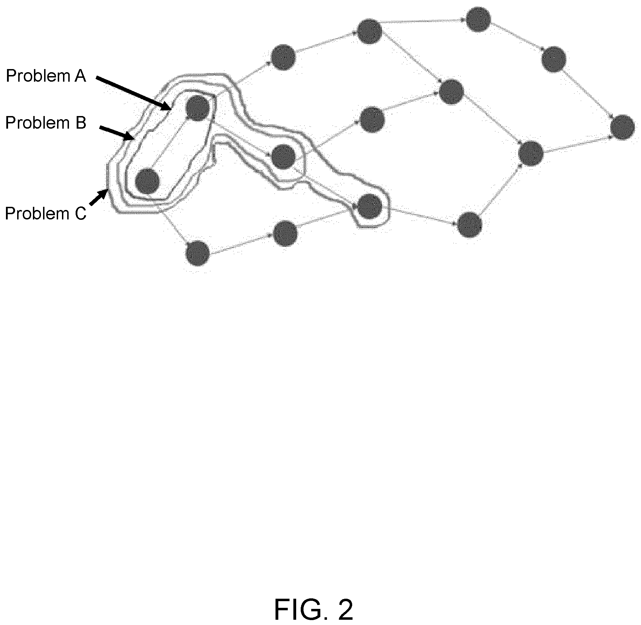

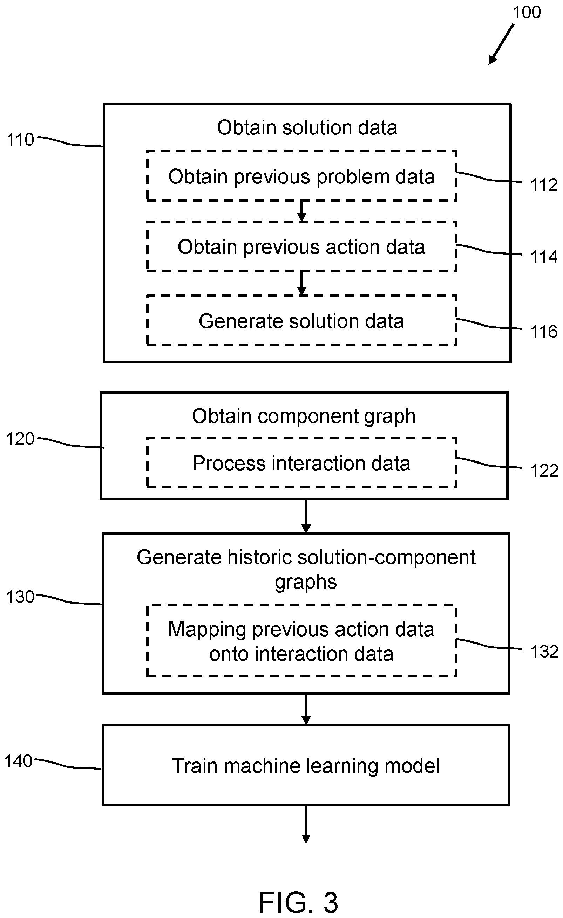

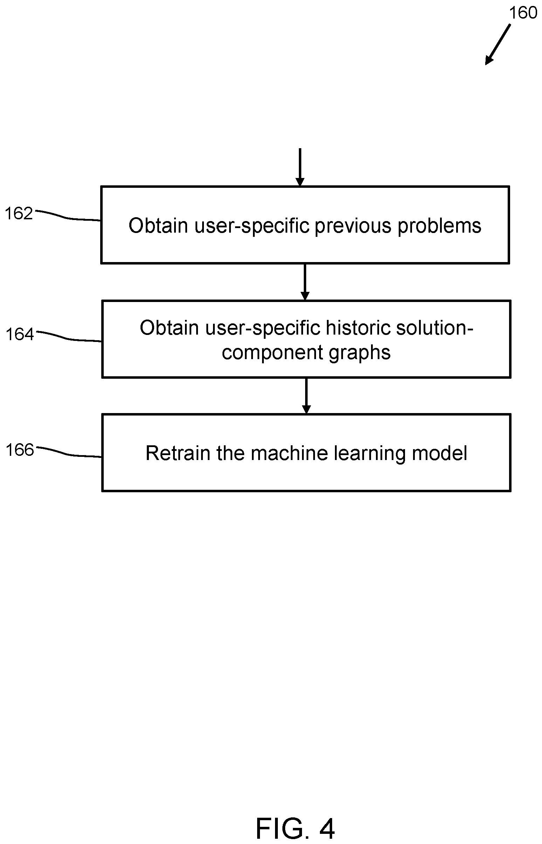

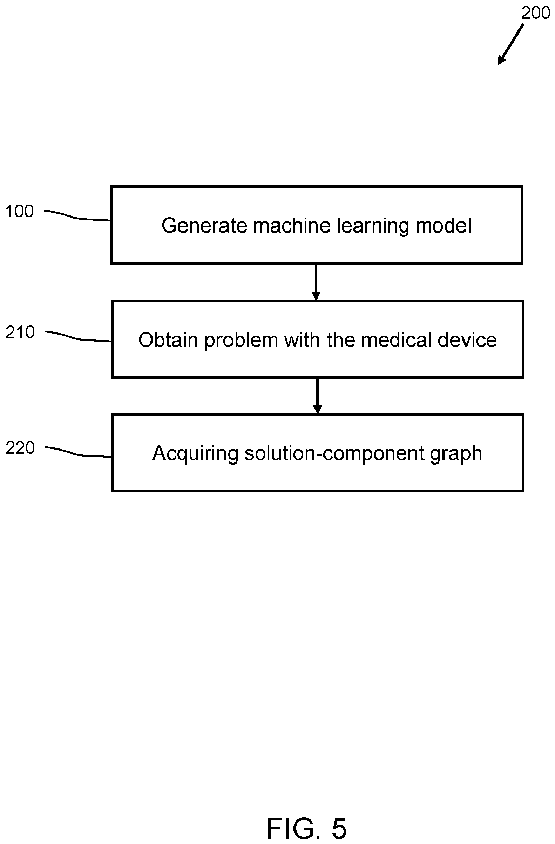

Fig. 1 presents a block diagram of a three-part system for determining the solvability of a problem with a medical device by a one or more individuals according to an embodiment of the invention; Fig. 2 is an example of a solution-component graph; Fig. 3 is a flow diagram of a method for training a machine learning model adapted to output a solution-component graph of a medical device according to an embodiment of the invention; Fig. 4 is a flow diagram of a method for re-training a machine learning model according to an aspect of another embodiment of the invention; Fig. 5 is a flow diagram of a method for generating a solution-component graph according to a further embodiment of the invention;

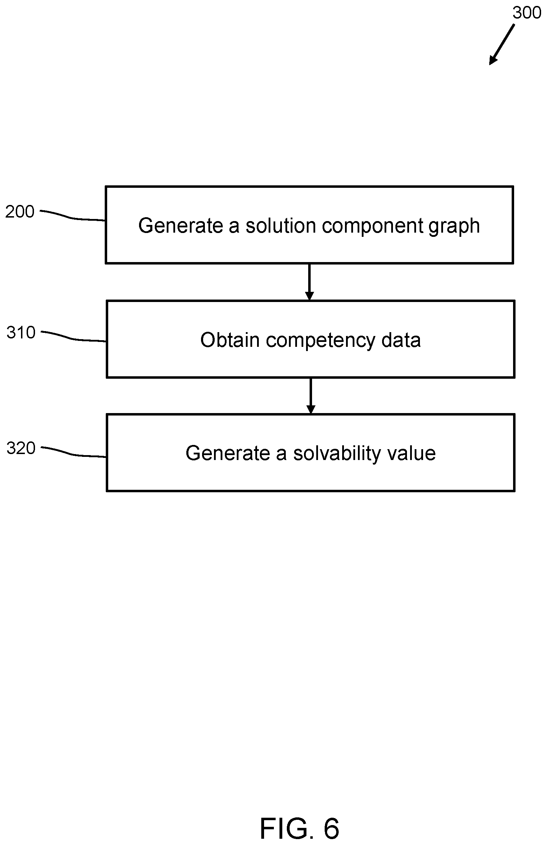

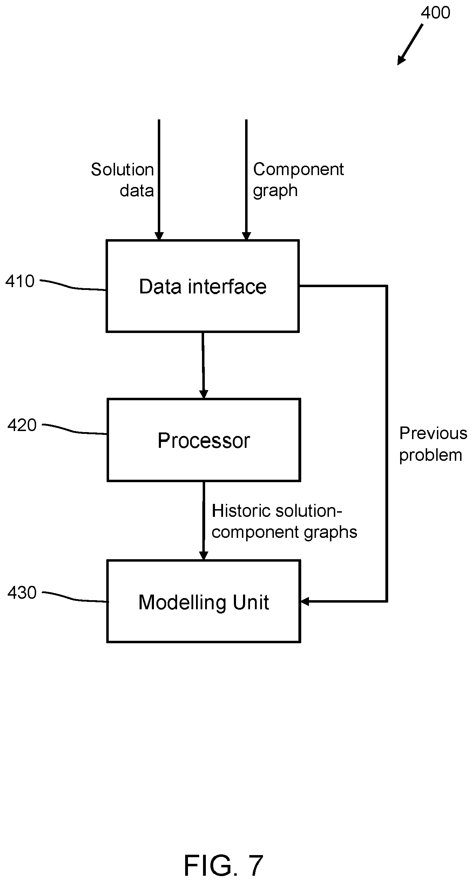

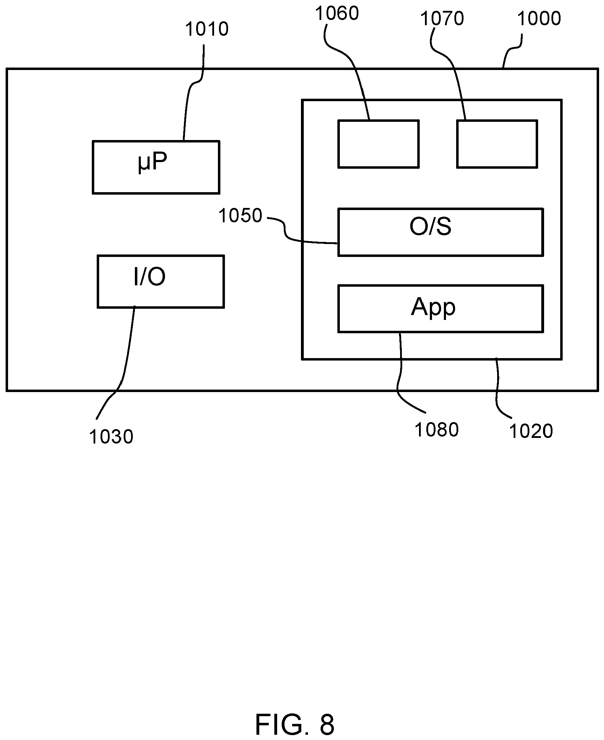

Fig. 6 is a flow diagram of a method for determining the solvability of a problem with a medical device by a one or more individuals according to another embodiment of the invention; Fig. 7 presents a simplified block diagram of a system for training a machine learning model adapted to output a solution-component graph of a medical device according to an additional embodiment of the invention; and Fig. 8 provides a simplified block diagram of a computer within which one or more parts of an embodiment may be employed.

DETAILED DESCRIPTION OF THE EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

The invention proposes concepts for aiding and/or improving the assessment of problems with medical devices. Specifically, solution-component graphs are generated from solution data (of previously resolved problems) and a component graph (representing interactions between components of the medical device). From these solution-component graphs, an understanding of how actions performed on components of the medical device provide a solution for a problem with the medical device is obtained. The invention utilizes the solution-component graphs to train a machine learning model, such that a solution-component graphs for new problems can be generated.

Such solution-component graphs map actions that a user (i.e. a technician or an engineer) may take on components of the medical device in order to diagnose and resolve a problem. As this may be obtained from a simple statement of a problem using the machine learning model trained according to embodiments of the invention, the invention essentially provides a simple way in which a list of (potential) actions needed to resolve the problem. These actions can be compared to previous actions taken by parties (and a summary of a party's competencies) in order to determine whether the party are capability of performing the actions. Thus, an appropriate allocation of work to enact a solution to the problem may be performed - saving time and money.

In other words, embodiments of the invention provide concepts for generating a machine learning model adapted to output a solution-component graph representing possible actions to be performed on components of a medical device to solve a problem with the medical device are proposed. Solution-component graphs for (new and previously seen) problems with the medical device can be generated, and can be compared to competencies of one or more individuals in order to ascertain the likelihood that the individual(s) can perform the actions to solve the problem. Thus, embodiments of the invention provide an efficient and accurate means for managing the process of diagnosing and fixing a medical device.

The crux of the invention lies in the training of a machine learning model that can generate a solution-component graph representing a sequence of actions performed on components of a type of medical device to solve a (new) problem. This can then be used to automatically assess the competency of groups (i.e. field service engineers, in-house biomedical engineers, etc.) to solve the problem.

For example, biomedical engineers employed by health facilities have at least basic knowledge of the medical devices within the medical facility, enabling them to solve certain problems with the medical devices. However, for many problems there is a reliance on Original Equipment Manufacturers (OEMs) to diagnose the problem and implement a solution. Typically, OEM service engineers diagnose/fix the problem using remote access, or may visit the healthcare facility (i.e. when replacement of parts is required). This process consumes a lot of time and in certain cases increases the downtime of the medical device.

Embodiments of the problem overcome some of the problems with pre-existing diagnostic and resolution proves by the automatic and accurate assessment as to whether a (new, as well as previously seen) problem can be solved by the in-house team of biomedical engineers, or with the aid of OEMs. More generally, embodiments provide an assessment of a competency/capability of parties (i.e. one or more individuals) in solving a prescribed problem. By this approach, (even before reporting the issue to OEMs) the bio-medical engineers may solve the issue by identifying the sub-parts of the new problem which are similar to the problems solved in the past. In some embodiments, there may be provided a solvability value indicating how far/to what extend one or more individuals can solve the problem (i.e. an individual may be able to diagnose an issue, but not implement the full solution). Overall, this solution helps to minimise the involvement of OEMs by optimizing the work, thus saving time of skilled technicians to work on more complex issues.

In this way, a downtime of the medical device is reduced by facilitating a quicker solution to the problem - resulting in better medical device customer satisfaction.

Put another way, currently it is the case that each time a new issue arises with a medical device, assistance of an OEM is required to solve the issue. This results in higher down time of the medical device at the medical facility. Moreover, there is a higher workload for field service engineers (FSEs) of the OEM, due to limited knowledge of whether some parts of the new problem can be solved by biomedical engineers at the medical facility without the involvement of FSEs. Thus, current processes result in lower customer engagement and satisfaction.

These problems are resolved by embodiments of the present invention by the identification of parts (or sub parts) of the problem that one or more individuals can solve. Specifically, this is achieved by generating of component-solution graphs for new problems, which can be compared to known competencies of individuals, in order to assess an extent to which the individual can find a resolution to the problem.

A simplified system for facilitating the above is shown in Fig. 1. The system may be considered in three parts/phases: a global training phase, an adapt phase and a site specific inference phase. Each of these phases are explained below, in detail.

The global training phase is for producing a machine learning model (in this case a generative adversarial network, GAN) for a (type of) medical device. The machine learning model may be for one make and model of a medical device. Essentially, the machine learning model is capable of generating solution-component graphs (i.e. a graph representing possible actions to be performed on components of the medical device to solve a problem) based on a problem input to the machine learning model.

The global training phase may comprise the main components of a problem to solution mapper, a problem to solution mapper on a component graph, a GAN model training (problem to component graph coverage), and a component graph.

The problem to solution mapper generates solution data by mapping a reported problem (i.e. a problem reported by a customer) to a corresponding solution. The mapper takes a problem statement, device log, service manual and service history as input parameters. Each of the problems is mapped to a corresponding solution(s) coming from either the service history or the service manual. The problem statement is also mapped to device log data corresponding to time duration when problem/issue was observed.

It is noted that it is likely that there are many similar problems and similar solutions. Thus, in order to reduce redundancy in the solution data a cosine similarity matrix is used, represented by:

$$\cos(\theta) = \frac{A \cdot B}{\|A\|\|B\|} = \frac{\sum_{i=1}^{n} A_i B_i}{\sqrt{\sum_{i=1}^{n} A_i^2} \sqrt{\sum_{i=1}^{n} B_i^2}}$$

Where A is vector representing one problem, and B a vector of another problem. A high value threshold is used to determine whether two problem statements are similar or not. If similar, the problems are merged, or one is removed, to avoid redundancy. Ultimately the solution data may be represented in a table for, such as:

| Problem Statement | Solution | Log Data |
|---|---|---|
| Problem 1 | Solution A | Data 1 |
| Problem 2 | Solution B | Data 2 |

In one embodiment, (historic) solution data is gathered, comprising the associated problem, solution and log data. A vocabulary of distinct words can be generated using this data. For each word, a vector representation is determined and used to calculate cosine similarity. For this, pre-trained word embedding vectors can be utilized, or a neural network may be used to extract the word embeddings.

Once all word embeddings are gathered, a sentence can be taken and used to create a word embedding for that sentence by averaging word vectors for all words in that sentence. Similarly, we can obtain word vectors for all problems in the solution data. Once we have these vectors, we can compute the cosine similarity which will result in identifying the problems which are closer, or which have similar context.

The above may be repeated to obtain the vector representation for all solution data (problems, solutions and log data).

Moving on, a component graph is built that represents interactions and communications of components in/of the medical device. In order to build this graph information from design documents, design and production drawings, and service manuals are used. The documents and drawings are parsed to extract component details and their interaction with other components. The information parsing and extraction is performed using natural language processing (NLP) based AI techniques

More specifically, each of the components is a node in a graph with stored information about itself, and the connection/relation it has with other components. These nodes are disjoint initially, a random node is selected for processing then it parses the connection/relation details and search is initiated for connected/related node. Once the connected node is found the connection is established. This process is performed for all connected nodes until a full component graph is generated.

The problem to solution mapper on component graph then uses the solution data and the component graph to create a solution-component graph. Essentially, the solution data is linked/overlayed with the component graph. This is achieved by parsing the solution data, and finding matching keywords to nodes of the component graph. Finding of keywords is achieved using NLP techniques, while the matching is carried out using semantic matching techniques. Each edge of the solution-component graph is given weight when there is matching text in the solution data. The magnitude of weight of each edge is dependent upon the number of times and relevance of the mention. The final solution-component graph that is mapped to problem shall have connections with greater than or equal to 1 weight for all the edges where there is reference, and weight of zero for the edges where there is no reference.

The problem associated with the solution data used to generate the solution-component graph is then used for building a machine learning model.

At the GAN model training block, a machine learning model (a GAN model) is trained so that for any given new solution-component graph can be generated. For model training, the problem statement and associated solution-component graph is provided. This trained machine learning model is further used for fine tuning and for inference in final phase.

In the adapt phase, the generic GAN model generated using the above method is re-trained to generate a site-specific model. This is useful for medical facilities that have different types of problems. The re-training of the model is achieved by using that particular sites log data, historical problem statements and historical service actions. This makes the re-trained model tuned to that particular medical facility. However, as the global trained model is used as a base, it keeps the essence of the other problems with it. This mechanism helps in getting better outcome when a new problem is faced at the medical facility, a better solution-component graph gets generated due to usage of global model as base.

At the user-specific inference phase, a new problem with the medical device is provided to the trained machine learning model. The trained model generates the solution-component graph for this new problem.

The solvability predictor compares the solution-component graph, historic solution-component graphs and competency data of one or more individuals to determine a solvability value. In some embodiments, the solvability predictor compares both the solution-component graphs and identifies the overlapping portions of the graph by traversing the nodes of the graph. Based on the intersection of the nodes the percentage of overlapping components of graph can be determined. From these overlapping nodes and competency data, an inference as to whether the new problem can be solved by a certain individual(s) can be determined (i.e. whether biomedical engineers can solve it, or whether OEM advice is required).

In specific embodiments, by identifying the overlapping portions, we can infer whether the new problem can be solved completely by the biomedical engineers, or what percentage of the problem requires OEM assistance. This information may then be used to inform a recommendation.

An example is shown in Fig. 2. In Fig. 2 there are 3 problems presented on a solution-component graph (A, B and C). The steps required to solve previous problems A and B are marked on the solution-component graph. The aim is to identify whether new problem C can be solved by a biomedical engineer team, if it must be solved jointly with an OEM team, or only by an OEM team. After completing the model training and site-specific inferencing we results are obtained from a recommendation engine.

For example, when it is predicted that there is a 0% overlap between the competency of the biomedical engineer team, the OEMs are contacted to solve the problem. When it is predicted that there is 100% overlap, the problem can be solved by the biomedical engineer team with no assistance required from OEM team. When it is predicted that there is a 75% overlap, the bio-medical engineer team can solve the overlapping steps (as shown in Fig. 2), and they should contact the OEM for the remaining steps.

By way of illustrative example, three actions are represented by the edges. (1) Replace the inverter board; (2) perform software based inverter fix; and (3) Observer logs generated during software-based inverter fix for failed inverter location. If the biomedical engineer team knows how to use software tool to analyze the problem, they also know how to observe the log file generated during software but not necessarily replacing the inverter. When an inverter problem is reported, and it requires a software based inverter fix, and the biomedical engineering team has solved the problem using the software tool in the past, then it can be solved by the biomedical team itself. However, if it needs to be replaced, them the support of the OEM is required.

Of course, we can keep track of competencies of each biomedical engineering team (and individuals within the team) by learning the competency by the type of problems they have solved in the past (or by user input). Once prediction results are obtained from the recommendation system, we may update the competency data.

For example, consider a situation in which the biomedical engineering team is competent in solving problems of consistent errors/warnings, and problems with tubes in some medical device. There is a problem identified in the past for which the solution is to solve the consistent errors/warnings, and a problem with tubes. For a new problem, there is generated solvability. Suppose there is a 66% overlap with the solution, and the previously unsolved part which is the problem with carbon brushes needs to be solved along with consistent errors/warnings, and problem with tubes. It can be concluded that the biomedical engineer team could solve the problem of carbon brushes as well. Thus, in this situation we can update this new identified problem which can be solved by the bio-medical engineer to the competency data.

Fig. 3 is a flow diagram of a method 100 for training a machine learning model adapted to output a solution-component graph of a medical device. The machine learning model is neural network (NN) based, and may preferably be generative adversarial network (GAN) based. The machine learning model is adapted to output, based on inputting a (new) problem with a medical device, the solution-component graph corresponding to the problem.

The solution-component graph represents possible actions to be performed on components of the medical device to solve (i.e. diagnose, fix, etc.) the problem of the medical device. In an embodiment, the solution-component graph has nodes representing actions to performed on components of the medical device, and edges connecting nodes, which when traversed potentially lead to a solution to the problem with the medical device.

Moreover, the medical device refers to one type (i.e. make and/or model) of medical device. For example, the medical device may be a CT scanner, an MRI machine, an X-Ray machine, etc. or may be a specific type of CT scanner, MRI machine, X-ray machine, etc.

At step 110, solution data describing sequences of previous actions performed to solve a plurality of previous problems with the medical device is obtained. In other words, the solution data has a list of previous problems with the medical device that have since been diagnosed, and resolved, along with corresponding actions (i.e. actions performed by a technician/user) to reach the diagnosis and resolution.

Ideally, the previous problems would encompass all possible problems with the medical device. However, it is often the case that previously unseen (or seemingly unseen) problems arise. Thus, the solution data simply comprises solutions to many problems that the medical device may experience.

The solution data may be obtained from a known database of solution data, and thus simply obtained. However, in some cases the solution data may need to be compiled. One embodiment of this is presented in (optional) sub-steps 112-116.

At sub-step 112, previous problem data describing historic issues with the medical device is obtained. The previous problem data may be obtained from problem statements and device logs associated with the medical device. Essentially, this contains a list of many of the problems experienced by the medical device in the past (i.e., not turning on, a certain error on a UI, etc.). This may be readily attainable from problem statements and device logs.

At sub-step 114, previous action data describing actions performed to solve historic issues with the medical device is obtained. The previous action data may be obtained from service histories associated with the medical device. For each of the historic issues identified in step 112, previous action data is obtained. This may be based on service histories and system logs corresponding to the time in which the problem in which the previous problem arose/was noted.

At sub-step 116, the previous problem data and previous action data is processed to generate the plurality of solution data. This may include actions associated with a resolution of the medical device (i.e. matching by time and/or labelling). Furthermore, the previous problem data and previous action data may be pre-processed to reduce redundancy in the data (i.e. similar problems and/or sequences of actions that have been conducted many times previously).

Specifically, processing the previous problem data and previous action data may include analysing the previous problem data and previous action data to generate raw solution data. Then, similar solutions are identified within the raw solution data based on the previous problem data and the previous action data. Accordingly, the plurality of solution data is generated based on the raw solution data and the identified similar solutions.

Next, at step 120, a component graph describing interactions between a plurality of components of the medical device is obtained. In other words, the component graph presents components of the medical device as nodes, and interactions (and communications) between the components are represented as edges.

The component graph may simply be obtained from a database, or may be generated. One method of generation is demonstrated in (optional) sub-step 122.

Specifically, interaction data of each component of the medical device is processed to generate the component graph. The interaction data describes an interactions/communications of said component with other components of the medical device. Thus, each interaction/communication can be used to link one component with another, thus forming a graph of nodes (components) connected by edges (interactions/communications)

The interaction data may be obtained from design documents, design drawings, production drawings and service manuals associated with components of the medical device.

At step 130, the solution data and the component graph are processed to generate a plurality of historic solution-component graphs. Put another way, each piece of solution data (relating to one problem) is interpreted in the light of the component graph in order to produce a solution-component graph indicating potential actions performed on components to resolve a problem.

One method of doing this is shown in sub-step 134. In step 134, a historic solution-component graph is be generated (with the step repeated for each problem encompassed by the solution data). Specifically, one of the sequences of previous actions described by the solution data is mapped onto the interactions between the components of the medical device described by the component graph. From this mapping, a historic solution-component graph may be generated.

On way to perform the mapping is to identify components identified within the sequences of previous actions described in the solution data by a natural language processing (NLP) algorithm. When a component/interaction is mentioned within the sequences of previous actions, the corresponding component/interaction may be found within the component graph. In this way, the solution data can be mapped onto the component graph to produce a solution-component graph.

At step 140, the previous problems and historic-solution-component graphs are provided to a neural network (NN)-based machine learning algorithm. The machine learning algorithm is trained adapted to output, based on inputting a (new) problem with a medical device, the solution-component graph corresponding to the problem.

The structure of an artificial NN (or, simply, neural network (NN)) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs), recurrent neural networks (RNNs), generative adversarial networks (GANs). In the present invention, the machine learning model is preferably a GAN. Given a training set, a GAN learns to generate new data with the same statistics as the training set.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

A core idea of a GAN is training through a discriminator, being a different neural network that assess the output of the GAN. The discriminator itself is dynamically updated, such that the GAN is not trained to minimize the distance to a specific known output. Instead, the GAN is trained to present a satisfactory output to the discriminator. This enables the GAN to learn in an unsupervised manner.

The training input data entries for the machine learning algorithm used in method 100 correspond to obtained previous problems. The training output data entries correspond to generated solution-component graphs. That is, the machine learning algorithm is trained using a training algorithm configured to receive an array of training inputs and respective known outputs, wherein a training input comprises previous problems and respective known output comprises solution-component graphs. In this way, the machine learning algorithm is trained to output a solution-component graph for an input of a (new) problem.

The method 100 may then proceed to retraining steps in order to adapt the machine learning model for a specific user. Indeed, this is shown in Fig. 4, which has a flow diagram of a method 160 for re-training a machine learning model according to an aspect of another embodiment of the invention. The user may be, for example, a healthcare facility, a department of a healthcare facility, or a group of healthcare facilities.

At step 162, user-specific previous problems are obtained. The user-specific previous problems describe historic issues with the medical device associated with the user. These are similar to the previous problems obtained in the step 110 of method 100, but are related to the user (i.e. relate to problems experienced by medical devices that the user owns/manages.

At step 164, user-specific historic solution-component graphs are obtained. The user-specific historic solution-component graphs are associated with the user-specific previous problems. These historic solution-component graphs may be similar to those generated in step 130 of method 100. However, they relate to the user-specific problems. These may be obtained from a database, or generated like in the method described in relation to steps 110-130 of method 100.

At step 166, the machine learning model is re-trained using a training algorithm configured to receive an array of training inputs and respective known outputs. In this case, a training input comprises one of the user-specific previous problems. A respective known output comprises the user-specific historic solution-component graph associated with the user-specific previous problem.

The training algorithm may be the same as that described in relation to step 140, with different training inputs and known outputs. Conversely, a different training algorithm may be used that is better suited for (re)training of the machine learning model.

Fig. 5 is a flow diagram of a method 200 for generating a solution-component graph according to a further embodiment of the invention. The solution-component graph represents possible actions to be performed on components of the medical device to solve a problem (i.e. a problem presented by a user).

At step 100, a machine learning model is generated. This step may be the same as any method described in relation to Fig. 1. Alternatively, the machine learning model may be a pre-generated machine learning model, and obtained from an appropriate memory. In any case, the machine learning model is adapted to output, based on inputting a (new) problem with a medical device, the solution-component graph corresponding to the problem.

At step 310, a problem describing an issue with the medical device is obtained. The problem may be in the form of a problem statement understandable by the machine learning model. Alternatively, this step may comprise modifying the problem to be recognizable/processable by the machine learning model. In specific embodiments, the problem is provided by (i.e. obtained from) a user (i.e. a person discovering the issue).

At step 320, the solution-component graph is acquired based on inputting problem to the generated machine learning model. As the machine learning model is trained based on previous problems and known solution-component graphs, it is adapted to produce a solution-component graph based on the problem input to it.

Thus, from this method, a solution-component graph may be obtained based on any problem related to the medical device. This is particularly useful for understanding how to reach a solution to a problem that has not been previously encountered by a user. Accordingly, an appropriate technician may be contacted for fixing of the medical device.

Fig. 6 is a flow diagram of a method 300 for determining the solvability of a problem with a medical device by a one or more individuals. In other words, the method provides a means for determining an extent to which a party (i.e. one or more individuals) can solve (i.e. diagnose, resolve and/or fix) a problem by utilizing aspects of the methods described in relation to Fig. 3-6. In particular, this may apply for determining whether in-house biomedical engineering team can solve a problem, or whether they need assistance from an OEMs service team.

At step 300, a solution-component graph is generated. The solution-component graph represents possible actions to be performed on components of the medical device to solve the problem. This is generated according to the method 300 described in reference to Fig. 5.

At step 410, competency data is obtained. The competency data describes an ability of the one or more individuals to perform a plurality of actions on the components of the medical device. In other words, the competency data describes the skills of the one or more individuals for solving problems by enacting steps. For example, one set of individuals may be able to run diagnostic software and replace minor parts, whole another set of individuals may be able to perform large-scale renovations of the medical device. Of course, the competency data may relate to individuals, or the abilities of a whole group.

The competency data may be derived from a history of previous problems solved and actions taken. Further, the competency data may be based on information input by the individuals themselves or on their behalf.

At step 420, the historic solution-component graphs, the solution-component graph and the competency data are processed to generate a solvability value. The solvability value is indicative of a likelihood that the one or more individuals can solve the problem with the medical device.

The solvability value may be provided, for example, in the form of a percentage chance that the whole problem can be solved by the one or more individuals, as a percentage of the number of possible actions that the one or more individuals can perform compared to the total number of actions. Furthermore, specific actions that the one or more individuals can be perform may be provided.

The solvability value is based on the historic solution-component graphs, the solution-component graph and the competency data. Essentially, actions performed in the past are compared to actions that are expected to be performed to solve the present problem. This can be contextualized with the competency data in order to ascertain the extent to which the one or more individuals can solve the problem, and thus provide a solvability value.

Fig. 7 presents a simplified block diagram of a system for training a machine learning model adapted to output a solution-component graph of a medical device. The system comprises a data interface 410, a processor 420, and a modelling unit 430.

The data interface 410 is configured to obtain solution data describing sequences of previous actions performed to solve a plurality of previous problems with the medical device. The data interface 410 is further configured to obtain a component graph describing interactions between a plurality of components of the medical device.

The data interface 410 may be any means capable of obtaining information (including the solution data and component graph). The data interface 410 may actively gather the information, or may simply receive the information that is input to it. The data interface 410 may further be configured to process data in order to generate the solution data and component graph, as described in reference to steps 110 and 120 of method 100.

Further, the processor 420 is configured to process the solution data and the component graph to generate a plurality of historic solution-component graphs.

Finally, the processor 420 provides the historic solution-component graphs, and the data interface 410 provides previous problem data to the training module. The training module is configured to train the machine learning model using a training algorithm configured to receive an array of training inputs and respective known outputs. The training input comprises one of the previous problems, and the respective known output comprises the historic solution-component graph associated with the previous problem.

Thus, the system 400 of Fig. 4 provides a means for training a machine learning model that can produce solution-component graphs for a (potential previously unseen) problem.

Fig. 8 illustrates an example of a computer 1000 within which one or more parts of an embodiment may be employed. Various operations discussed above may utilize the capabilities of the computer 1000. For example, one or more parts of a system for compressing raw medical image data for remote medical image reconstruction of an anatomy of a subject may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read- only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 3, 4, 5 and 6, and the system described in relation to Fig. 7, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for training a machine learning model adapted to output, based on inputting a problem with a medical device, a solution-component graph representing possible actions to be performed on components of the medical device to solve the problem, the method comprising:

   obtaining (110) solution data describing sequences of previous actions performed to solve a plurality of previous problems with the medical device;
   obtaining (120) a component graph describing interactions between a plurality of components of the medical device;
   processing (130) the solution data and the component graph to generate a plurality of historic solution-component graphs; and
   training (140) the machine learning model using a training algorithm configured to receive an array of training inputs and respective known outputs, wherein a training input comprises one of the previous problems, and wherein a respective known output comprises the historic solution-component graph associated with the previous problem.

2. The method of claim 1, wherein obtaining (110) the solution data comprises:

   obtaining (112) previous problem data describing historic issues with the medical device;
   obtaining (114) previous action data describing actions performed to solve historic issues with the medical device; and
   processing (116) the previous problem data and previous action data to generate the plurality of solution data.

3. The method of claim 2, wherein processing (116) the previous problems and sequences of previous actions comprises:

   analysing the previous problem data and previous action data to generate raw solution data;
   identifying similar solutions within the raw solution data based on the previous problem data and the previous action data; and

generating the plurality of solution data based on the raw solution data and the identified similar solutions.

4. The method of claim 2 or 3, wherein previous problem data is obtained from problem statements and device logs associated with the medical device, and previous action data is obtained from service histories associated with the medical device.

5. The method of any of claims 1-4, wherein obtaining (120) the component graph comprises processing (122) interaction data of each component of the medical device describing an interaction of said component with other components of the medical device, to generate the component graph.

6. The method of claim 5, wherein the interaction data is obtained from design documents, design drawings, production drawings and service manuals associated with components of the medical device.

7. The method of any of claims 1-6, wherein generating (130) each historic solution-component graph comprises:

mapping (132) one of the sequences of previous actions described by the solution data onto the interactions between the components of the medical device described by the component graph; and

generating the historic solution-component graph based on the mapping.

8. The method of claim 7, wherein the mapping is based on processing the sequences of previous actions and the components of the medical device using a natural language processing algorithm.

9. The method of any of claims 1-8, wherein the machine learning model is a generative adversarial network model.

10. The method of any of claims 1-9, further comprising retraining (160) the machine learning model for a user by:

obtaining (162) user-specific previous problems describing historic issues with the medical device associated with the user;

obtaining (164) user-specific historic solution-component graphs associated with the user-specific previous problems; and

retraining (166) the machine learning model using a training algorithm configured to receive an array of training inputs and respective known outputs, wherein a training input comprises one of the user-specific previous problems, and wherein a respective known output comprises the user-specific historic solution-component graph associated with the user-specific previous problem.

11. A method for generating a solution-component graph representing possible actions to be performed on components of the medical device to solve the problem, the method comprising:

generating (100) a machine learning model according to any of claims 1-10;

obtaining (210) a problem describing an issue with the medical device;

acquiring (220) the solution-component graph based on inputting problem to the generated machine learning model.

12. A method for generating a solution-component graph using a machine learning model trained to output, based on inputting a problem with a medical device, the solution-component graph representing possible actions to be performed on components of the medical device to solve the problem, the method comprising:

obtaining a problem describing an issue with the medical device;

acquiring the solution-component graph based on inputting problem to the generated machine learning model.

13. A method for determining the solvability of a problem with a medical device by a one or more individuals, the method comprising:

generating (200) a solution-component graph representing possible actions to be performed on components of the medical device to solve the problem according to claim 11 or 12;

obtaining (310) competency data describing an ability of the one or more individuals to perform a plurality of actions on the components of the medical device;

processing (320) the historic solution-component graphs, the solution-component graph and the competency data to generate a solvability value indicative of a likelihood that the one or more individuals can solve the problem with the medical device.

14. A computer program comprising computer program code means adapted, when said computer program is run on a computer, to implement the method of any of claims 1-13.

**15.** A system for training a machine learning model adapted to output, based on inputting a problem with a medical device, a solution-component graph representing possible actions to be performed on components of the medical device to solve the problem, the system comprising:

a data interface (410) configured to:

obtain solution data describing sequences of previous actions performed to solve a plurality of previous problems with the medical device;

obtain a component graph describing interactions between a plurality of components of the medical device;

a processor (420) configured to process the solution data and the component graph to generate a plurality of historic solution-component graphs; and

a training module (430) configured to train the machine learning model using a training algorithm configured to receive an array of training inputs and respective known outputs, wherein a training input comprises one of the previous problems, and wherein a respective known output comprises the historic solution-component graph associated with the previous problem.

**Global Training Phase**

Problem (customer complaint) ⇨ Problem to solution mapper ⇨ Problem to solution mapper on component graph ⇨ GAN model training

Device Log ⇨

Service History

Service History

Component Graph

**User Specific Inference Phase**

Recommendation ← Solvability Predictor ⇦ Trained Model ⟸

Component Graph Coverage

Historical Component Graph

New Problem

**Adapt Phase**

User Specific GAN model re-training

Log Data

Historical Problems

Service History

FIG. 1

Problem A

Problem B

Problem C

FIG. 2

100

110 —

**Obtain solution data**

- - - - - - - - - - - - - - - - - -
Obtain previous problem data — 112
- - - - - - - - - - - - - - - - - -

↓

- - - - - - - - - - - - - - - - - -
Obtain previous action data — 114
- - - - - - - - - - - - - - - - - -

↓

- - - - - - - - - - - - - - - - - -
Generate solution data — 116
- - - - - - - - - - - - - - - - - -

120 —

**Obtain component graph**

- - - - - - - - - - - - - - - - - -
Process interaction data — 122
- - - - - - - - - - - - - - - - - -

130 —

**Generate historic solution-component graphs**

- - - - - - - - - - - - - - - - - -
Mapping previous action data onto interaction data — 132
- - - - - - - - - - - - - - - - - -

140 —

**Train machine learning model**

FIG. 3

160

162 — Obtain user-specific previous problems

164 — Obtain user-specific historic solution-component graphs

166 — Retrain the machine learning model

# FIG. 4

200

| Generate machine learning model |
|---|

100

| Obtain problem with the medical device |
|---|

210

| Acquiring solution-component graph |
|---|

220

# FIG. 5

FIG. 6

400

Solution data | Component graph

410 — Data interface

420 — Processor

Historic solution-component graphs

Previous problem

430 — Modelling Unit

FIG. 7

FIG. 8

# EUROPEAN SEARCH REPORT

**Application Number**

EP 22 20 7547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/410163 A1 (SHAH TAPAN [IN] ET AL) 31 December 2020 (2020-12-31) * paragraphs [0001] - [0031]; figures 1A, 1B * ----- | 1-15 | INV. G06N3/09 G16H40/40 G06Q10/20 |

**TECHNICAL FIELDS SEARCHED (IPC)**

G06N
G16H
G06Q
G06F
G05B

The present search report has been drawn up for all claims

| Place of search | Date of completion of the search | Examiner |
|---|---|---|
| The Hague | 28 April 2023 | Nourestani, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 7547

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2023

| Patent document cited in search report | | Publication date | Patent family member(s) | | Publication date |
|---|---|---|---|---|---|
| US 2020410163 | A1 | 31-12-2020 | CN | 111819584 A | 23-10-2020 |
| | | | EP | 3759662 A1 | 06-01-2021 |
| | | | US | 2020410163 A1 | 31-12-2020 |
| | | | WO | 2019169147 A1 | 06-09-2019 |

EPO FORM P0459

For more details about this annex : see Official Journal of the European Patent Office, No. 12/82